# EUROPÄISCHE PATENTANMELDUNG

(11) **EP 2 236 103 A1**
(43) Veröffentlichungstag der Anmeldung: **06.10.2010**
(21) Anmeldenummer: 09156710.7
(22) Anmeldetag: 30.03.2009
(51) Int. Cl.: A61B 19/00, G06F 1/32

(54) **Verfahren zur Stromversorgung eines medizinischen Gerätesystems**

(71) Anmelder: Dräger Medical AG & Co. KG, 23558 Lübeck (DE)
(72) Erfinder: Becker, Dr., Uwe, 82223, Eichenau (DE)
(74) Vertreter: Strauss, Steffen

(57) **Zusammenfassung**

Bei einem medizinisches Gerätesystem (1), umfassend wenigstens ein medizinisches Gerät (6), z. B. ein Beatmungsgerät (7), einen Patientenmonitor (8), ein Anästhesiegerät, ein Inkubator und/oder ein Bildschirm (9), eine Zentraleinheit (10) zur Stromversorgung des wenigstens einen medizinischen Gerätes (6), wenigstens ein Mittel (11) zur elektrischen Verbindung der Zentraleinheit (10) mit dem wenigstens einen medizinischen Gerät (6), soll die medizinischen Geräte (6) zentral ein- und ausgeschaltet werden können und keine Kosten bei den medizinischen Geräten (6) für die Stromversorgung durch eine Batterie (5) oder einen Gleichrichter (4) anfallen. Das medizinische Gerätesystem (1) soll in der Herstellung preiswert sein und einen sichereren und zuverlässigen Umgang ermöglichen.

Diese Aufgabe wird dadurch gelöst, dass die Zentraleinheit (10) eine Batterie (5) und/oder einen Gleichrichter (4) aufweist, so dass das wenigstens eine medizinische Gerät (6) von der Zentraleinheit (10) mit Gleichstrom versorgbar ist.

## Beschreibung

Die vorliegende Erfindung betrifft ein medizinisches Gerätesystem gemäß dem Oberbegriff des Anspruches 1 und ein Verfahren zur Stromversorgung eines medizinischen Gerätesystems gemäß dem Oberbegriff des Anspruches 9.

In Intensivstationen oder am Bett von Patienten werden verschiedene medizinische Geräte eingesetzt, z. B. ein Beatmungsgerät und ein Bildschirm, um den Patienten medizinisch zu versorgen. Die medizinischen Geräte benötigten Gleichstrom im Allgemeinen mit einer Niederspannung von 24 V zum Betrieb. Aus diesem Grund ist es erforderlich, dass jedes einzelne medizinische Gerät einen Gleichrichter und eine Batterie aufweist. Die medizinischen Geräte werden dabei an eine Wechselspannungsquelle, z. B. an das Stromnetz des Krankenhauses mit einer Spannung von 220 V, angeschlossen. Mittels des Gleichrichters wird der notwendige Gleichstrom für den Betrieb des medizinischen Gerätes zur Verfügung gestellt. Eine Batterie in dem medizinischen Gerät dient dazu, bei einem Stromausfall der Wechselspannungsquelle für kurze Zeit die Stromversorgung des medizinischen Gerätes sicherzustellen.

In nachteiliger Weise ist es daher erforderlich, beispielsweise in einer Intensivstation von jedem einzelnen medizinischen Gerät ein Netzkabel zu der Wechselspannungsquelle zu legen. Insbesondere bei beengten Raumverhältnissen können diese Netzkabel die Arbeit in der Intensivstation behindern. Außerdem ist es erforderlich, jedes medizinische Gerät einzeln ein- und auszuschalten, so dass dadurch eine einheitlich Bedienung sämtlicher medizinischen Geräte eines medizinischen Gerätesystems nicht möglich ist.

Die DE 198 02 340 B4 zeigt ein medizinisches Gerätesystem mit einer Anschlusseinheit und medizinischen Geräten zur Diagnose und Therapie. Jedes der medizinischen Geräte umfasst eine Systemleitung, die mittels eines Systemsteckers an die Anschlusseinheit anschließbar ist. Die Anschlusseinheit weist Mittel auf zum Datenaustausch zwischen den medizinischen Geräten über die Systemleitung und die Versorgung der medizinischen Geräte mit Energie über die Systemleitung.

Aus der DE 199 07 538 C2 ist ein Kommunikationssystem mit Kommunikationsteilnehmern als medizinischen Geräten bekannt. Die Kommunikationsteilnehmer sind über wenigstens ein an einen Netzanschluss anschließbares Netzkabel miteinander elektrisch verbunden, über das dem Kommunikationsteilnehmern Energie zuführbar ist und über das zugleich von einem ersten zu einem zweiten Kommunikationsteilnehmer eine wenigstens zweikanalige Übertragung von Daten in Form von elektrischen Signalen erfolgen kann.

Die DE 198 02 341 C1 zeigt ein medizinisches System mit einer Basiseinheit, an die verschiedene medizinische Geräte zur Bildung verschiedener therapeutischer und/oder diagnostischer Arbeitsplätze anschließbar sind. Die Basiseinheit weist Mittel zur Steuerung des Datenaustausches zwischen der Basiseinheit und den medizinischen Geräten und zur Steuerung des Datenaustausches zwischen den medizinischen Geräten auf. Die Basiseinheit weist Mittel zur Anzeige von mittels der medizinischen Geräte gewonnenen Informationen auf. Der Anschluss der medizinischen Geräte und eines Monitors an die Basiseinheit zur Bildung des Arbeitsplatzes erfolgt über eine Verbindungsleitung. Die Verbindungsleitung weist zwei Kabel zur Übertragung von Daten, ein Videokabel zur Übertragung von Bilddaten und zwei Energieversorgungskabel auf.

Die Aufgabe der vorliegenden Erfindung besteht deshalb darin, ein medizinisches Gerätesystem und ein Verfahren zur Stromversorgung eines medizinischen Gerätesystems zur Verfügung zu stellen, bei dem die medizinischen Geräte zentral ein- und ausgeschaltet werden können und keine Kosten bei den medizinischen Geräten für die Stromversorgung durch eine Batterie oder einen Gleichrichter anfallen. Das medizinische Gerätesystem soll in der Herstellung preiswert sein und einen sichereren und zuverlässigen Umgang ermöglichen.

Diese Aufgabe wird gelöst mit einem medizinischen Gerätesystem, umfassend wenigstens ein medizinisches Gerät, z. B. ein Beatmungsgerät, ein Anästhesiegerät, ein Inkubator und/oder ein Bildschirm, eine Zentraleinheit zur Stromversorgung des wenigstens einen medizinischen Gerätes, wenigstens ein Mittel zur elektrischen Verbindung der Zentraleinheit mit dem wenigstens einen medizinischen Gerät, wobei die Zentraleinheit eine Batterie und/oder einen Gleichrichter aufweist, so dass das wenigstens eine medizinische Gerät von der Zentraleinheit mit Gleichstrom versorgbar ist.

In vorteilhafter Weise benötigen damit die medizinischen Geräte keine separaten Batterien und Gleichrichter mehr, so dass dadurch in den medizinischen Geräten die Batterien und der Gleichrichter eingespart werden können. Der Gleichrichter und die Batterie zur Notstromversorgung sind damit zentral in der Zentraleinheit angeordnet.

In einer weiteren Ausgestaltung sind die Batterie und/oder der Gleichrichter modulartig aufgebaut. An der Zentraleinheit können dadurch mittels entsprechender elektrischen Verbindungsmittel zusätzliche Batterien und/oder zusätzliche Gleichrichter angeordnet und verbunden werden, so dass dadurch die Zentraleinheit unterschiedliche elektrische Leistungen für verschiedene medizinische Gerätesysteme, d. h. für eine verschiedene Anzahl von medizinischen Geräten und/oder für medizinische Geräte mit unterschiedlicher elektrischer Leistungsaufnahme, möglich ist.

Insbesondere umfasst das medizinische Gerätesystem ein Netzkabel zum elektrischen Verbinden der Zentraleinheit mit einer Wechselspannungsquelle, z. B. im Bereich von 220 V oder 110 V, und/oder die Spannung des Gleichstromes zur Versorgung des wenigstens einen medizinischen Gerätes ist Niederspannung, insbesondere eine Spannung kleiner als 50 V oder kleiner als 30 V, z. B. im Bereich von 24 V.

In einer weiteren Ausgestaltung umfasst das wenigstens eine Mittel ein Bussystem und/oder wenigstens ein Verbindungskabel zur elektrischen Verbindung der Zentraleinheit mit dem wenigstens einen medizinischen Gerät und/oder zur Verbindung von wenigstens zwei medizinischen Geräten untereinander und/oder die Zentraleinheit und/oder wenigstens ein medizinisches Gerät weist ein Interface, z. B. einen Stecker, auf zur elektrischen Verbindung der Zentraleinheit mit dem wenigstens einen medizinischen Gerät und/oder zur Verbindung von wenigstens zwei medizinischen Geräten untereinander. Mittels des Bussystems und/oder des wenigstens einen Verbindungskabels kann das wenigstens eine elektrische Gerät mit der Zentraleinheit elektrisch verbunden werden. Dadurch kann das wenigstens eine medizinische Gerät mit elektrischer Energie aus der Batterie und/oder dem Gleichrichter der Zentraleinheit versorgt werden. Ferner können Daten von dem medizinischen Gerät zu der Zentraleinheit und/oder umgekehrt übertragen werden sowie auch Daten zwischen zwei medizinischen Geräten übermittelt werden.

In einer ergänzenden Ausführungsform sind Daten von der Zentraleinheit zu dem wenigstens einen medizinischen Gerät übertragbar und/oder umgekehrt und/oder Daten zwischen wenigstens zwei medizinischen Geräten sind übertragbar und/oder Daten sind als elektrische Signale übertragbar.

Vorzugsweise weist die Zentraleinheit und/oder das wenigstens eine medizinische Gerät wenigstens eine Anzeigeeinheit, z. B. ein Bildschirm und/oder wenigstens eine Leuchte, z. B. LED-Leuchte auf und/oder die Zentraleinheit weist eine Bedieneinheit, z. B. wenigstens einen Schalter und/oder eine Tastatur, auf. Die Anzeigeeinheit ist vorzugsweise ein gesondertes medizinisches Gerät, kann jedoch auch in die Zentraleinheit integriert sein. Die Bedieneinheit ist vorzugsweise in die Zentraleinheit integriert und dient zum zentralen ein- und ausschalten sowie zum Steuern sämtlicher medizinischer Geräte des medizinischen Gerätesystems.

In einer Variante sind an der Zentraleinheit wenigstens ein medizinisches Gerät, insbesondere sämtliche an der Zentraleinheit angeschlossenen medizinischen Geräte, ein- und/oder ausschaltbar und/oder bedienbar und/oder steuerbar und/oder Daten wenigstens eines medizinischen Gerätes sind ablesbar. In vorteilhafter Weise ist es damit nicht mehr erforderlich, die medizinischen Geräte des medizinischen Gerätesystems einzeln ein- und auszuschalten. Die medizinischen Geräte des medizinischen Gerätesystems können damit von nur einem zentralen Schalter an der Zentraleinheit oder einem beliebigen medizinischen Gerät ein- und ausgeschaltet werden.

Zweckmäßig weist wenigstens ein medizinisches Gerät keinen Gleichrichter und/oder keine Batterie auf, so dass dem wenigstens einen medizinischen Gerät elektrische Energie ausschließlich von der Zentraleinheit zuführbar ist und/oder ein in dieser Schutzrechtsanmeldung beschriebenes Verfahren ist ausführbar.

Erfindungsgemäßes medizinisches Gerät, z. B. ein Beatmungsgerät, ein Anästhesiegerät, ein Inkubator oder ein Bildschirm, das zum Betrieb, vorzugsweise ausschließlich, Gleichstrom benötigt, wobei das medizinische Gerät keinen Gleichrichter und/oder keine Batterie aufweist. Das medizinische Gerät ist damit in der Herstellung preiswert, weil kein Gleichrichter und keine Batterie erforderlich sind. Der Gleichrichter und die Batterie sind in der Zentraleinheit für das medizinische Gerät angeordnet.

Erfindungsgemäßes Verfahren zur Stromversorgung eines medizinischen Gerätesystems mit wenigstens einem medizinischen Gerät, einer Zentraleinheit zur Stromversorgung des wenigstens einen medizinischen Gerätes und wenigstens einem Mittel zur elektrischen Verbindung der Zentraleinheit mit dem wenigstens einen medizinischen Gerät, insbesondere eines in dieser Schutzrechtsanmeldung beschriebenes medizinischen Gerätesystems, und elektrischer Strom von der Zentraleinheit zu wenigstens einem medizinischen Gerät durch das wenigstens eine Mittel geleitet wird, wobei, vorzugsweise ausschließlich, Gleichstrom von der Zentraleinheit zu dem wenigstens einen medizinischen Gerät geleitet wird, insbesondere, vorzugsweise ausschließlich, zur Versorgung des wenigstens einen medizinischen Gerätes mit elektrischen Strom.

In einer weiteren Ausgestaltung wird in einem Gleichrichter in der Zentraleinheit Wechselstrom in Gleichstrom umgewandelt und/oder in einer Batterie in der Zentraleinheit wird elektrische Energie gespeichert.

Vorzugsweise wird Wechselstrom aus einer Wechselspannungsquelle, z. B. mit einer höheren Spannung von wenigstens 110 V oder 220 V, in Gleichstrom umgewandelt und/oder die höhere Spannung in Niederspannung, z. B. mit einer Spannung von weniger als 50 V oder 30 V, z. B. im Bereich von 24 V, transformiert.

In einer ergänzenden Variante wird Gleichstrom aus dem Gleichrichter und/oder aus der Batterie zu dem wenigstens einen medizinischen Geräte geleitet.

In einer weitern Variante übermittelt wenigstens ein medizinisches Gerät Daten, insbesondere hinsichtlich des elektrischen Leistungsbedarfes und/oder der Art oder der Priorität des wenigstens einen medizinischen Gerätes, zu der Zentraleinheit. Die Priorität des medizinischen Gerätes gibt an, wie wichtig das medizinische Gerät für den zu behandelnden Patienten ist. Bei einer hohen Priorität wird das medizinische Gerät vorrangig mit elektrischer Energie versorgt vor einem medizinischen Gerät mit einer niedrigeren Priorität. Wird von dem medizinischen Gerät die Art des medizinischen Gerätes an die Zentraleinheit übermittelt, kann die Zentraleinheit aus der Art des medizinischen Gerätes die Priorität innerhalb der medizinischen Geräte des medizinischen Gerätesystems ermitteln.

In einer weiteren Ausgestaltung wird, wenn die Summe des elektrischen Leistungsbedarfes von wenigstens zwei medizinischen Geräten größer ist als die elektrische Leistung der Zentraleinheit zur Versorgung wenigstens eines medizinischen Gerätes von der Zentraleinheit wenigstens ein medizinisches Gerät in einer absteigenden Reihenfolge der Priorität betrieben, so dass der elektrische Leistungsbedarf oder die Summe des elektrischen Leistungsbedarfes des wenigstens einen betriebenen medizinischen Gerätes kleiner ist als oder gleich ist wie die elektrische Leistung der Zentraleinheit zur Versorgung des wenigstens einen medizinischen Gerätes.

Damit ist sichergestellt, dass zunächst diejenigen medizinischen Geräte mit der höchsten Priorität zuerst mit elektrischer Energie versorgt werden und erst anschließend Geräte mit der niedrigeren Priorität. Sind beispielsweise als medizinische Geräte ein Beatmungsgerät, ein Anästhesiegerät und ein Monitor vorhanden, so wird das Beatmungsgerät und das Anästhesiegerät bevorzugt und zuerst mit elektrischer Energie versorgt und erst wenn genügend elektrische Energie zur Verfügung steht auch der Monitor mit elektrischer Energie versorgt. Dadurch kann z. B. bei einem Stromausfall, wenn die elektrische Energie ausschließlich aus der Batterie der Zentraleinheit zur Verfügung gestellt wird, auch eine medizinische sichere Versorgung des Patienten sichergestellt werden.

Insbesondere werden Daten, z. B. Statusinformationen, technische oder medizinische Daten, zu wenigstens einem medizinischen Gerät, vorzugsweise sämtlichen medizinischen Geräten, an der Zentraleinheit oder einem medizinischen Gerät angezeigt, z. B. an einem Bildschirm oder wenigstens einer Leuchte, z. B. LED-Leuchte, insbesondere werden Daten zu wenigstens zwei medizinischen Geräten an nur einer Anzeigeeinheit, z. B. Bildschirm angezeigt.

Statusinformationen sind beispielsweise Informationen oder Daten, ob ein medizinisches Gerät ein- oder ausgeschaltet ist oder ob es über eine Standby-Funktion mit niedrigem elektrischen Leistungsbedarf verfügt oder die Standby-Funktion aktiviert ist. Technischen Daten zu einem medizinischen Gerät sind Daten, ob beispielsweise das medizinische Gerät zusätzlich auch über eine Batterie verfügt oder welche elektrische Leistung das medizinische Gerät benötigt. Sofern das medizinische Gerät über eine Batterie verfügt, kann dieses an das medizinische Gerätesystem angeschlossen werden und es benötigt bei einem Betrieb mittels der in dem medizinischen Gerät angeordneten Batterie keine elektrische Energie aus der Zentraleinheit, so dass dadurch ein Betrieb dieses medizinischen Gerätes möglich ist, obwohl keine zusätzliche elektrische Leistung aus der Zentraleinheit zur Verfügung steht. Medizinische Daten betreffen beispielsweise Daten zum Patienten, die beispielsweise an einem Bildschirm graphisch dargestellt werden können.

In einer weiteren Ausgestaltung werden wenigstens ein medizinisches Gerät, insbesondere sämtliche an der Zentraleinheit angeschlossenen medizinischen Geräte, an der Zentraleinheit ein- und ausgeschaltet und/oder bedient und/oder gesteuert und/oder Daten wenigstens eines medizinischen Gerätes werden abgelesen.

Im Nachfolgenden wird ein Ausführungsbeispiel der Erfindung unter Bezugnahme auf die beigefügten Zeichnungen näher beschrieben. Es zeigt:

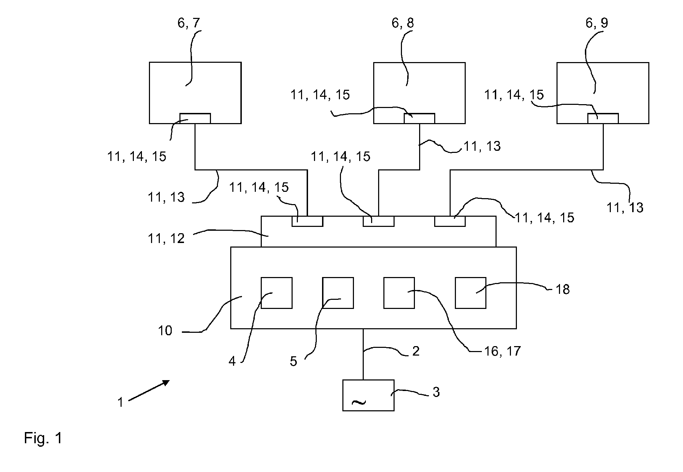

Fig. 1 eine stark schematisierte Darstellung eines medizinischen Gerätesystems.

In Fig. 1 ist stark schematisch ein medizinisches Gerätesystem 1 für eine Intensivstation eines Krankenhauses dargestellt. Die medizinischen Geräte 6 des medizinischen Gerätesystems 1 sind ein Beatmungsgerät 7, ein Patientenmonitor 8 und ein Bildschirm 9. Die medizinischen Geräte 6 dienen zur medizinischen und/oder therapeutischen Versorgung eines zu behandelnden Patienten in der Intensivstation (nicht dargestellt).

Die medizinischen Geräte 6 sind durch Mittel 11 zur elektrischen Verbindung elektrisch mit einer Zentraleinheit 10 verbunden. Die Mittel 11 umfassen ein Bussystem 12, Verbindungskabel 13 und Interface 14. Jedes medizinische Gerät 6 weist dabei ein Interface 14 auf, das eine erweiterte Funktionalität aufweist. Das Interface 14 der medizinischen Geräte 6 ist mit einer Logik ausgestattet, die auf einer Seite eine Kommunikation bzw. einen Datenaustausch mit dem Interface 14 anderer medizinischer Geräte 6 erlaubt. In dem Interface 14 ist außerdem ein Speicher vorhanden, in dem Daten, insbesondere technische Daten, zu dem jeweiligen medizinischen Gerät 6 gespeichert sind. Die technischen Daten sind beispielsweise Daten zur maximalen oder minimalen elektrischen

Leistungsaufnahme bzw. Abgabe sowie zur Art oder zu einer Priorität des medizinischen Gerätes 6. Ferner kann mittels der gespeicherten Daten in dem Interfache 14 das medizinische Gerät 6 von der Zentraleinheit 10 identifiziert werden.

In Fig. 1 ist nur je ein Verbindungskabel 13 von jeweils einem medizinischen Gerät 6 zu dem Bussystem 12 dargestellt. Dabei kann das Verbindungskabel 13 auch mehrere elektrische Leitungen enthalten. Die unterschiedlichen elektrischen Leitungen des Verbindungskabels 13 können dabei beispielsweise dazu dienen, entweder nur Daten in Form von elektrischen Signalen zu übertragen oder nur elektrischen Strom von der Zentraleinheit 10 zu den medizinischen Geräten 6 zu leiten. Zum Anschließen eines medizinischen Gerätes 6 an die Zentraleinheit 10 bzw. das Bussystem 12 der Zentraleinheit 10 ist das Verbindungskabel 13 mit einem Stecker 15 an einem entsprechend ausgebildeten Gegenstecker als Interface 14 an dem medizinischen Gerät 6 anzustecken. Ferner ist ein zweiter Stecker an dem Verbindungskabel 13 an einem Gegenstecker als Interface 14 des Bussystems 12 entsprechend anzustecken.

Die Zentraleinheit 10 umfasst einen Gleichrichter 4 und eine Batterie 5. Mittels eines Netzkabels 2 kann die Zentraleinheit 10 an eine Wechselspannungsquelle 3 angeschlossen werden. Die Wechselspannungsquelle 3 ist beispielsweise das Stromnetz eines Krankenhauses mit einer Spannung von 220 V. Der Gleichrichter 4 wandelt die Wechselspannung in eine Gleichspannung um und außerdem wird mittels eines Transformators (nicht dargestellt) die Spannung der Wechselspannungsquelle 3 auf eine Spannung von 24 V als Niederspannung herabgesenkt. Dieser damit an dem Gleichrichter 4 vorhandene elektrische Strom mit einer Spannung von 24 V wird sowohl zu der Batterie 5 als auch zu den medizinischen Geräten 6 mittels der Mittel 11 geleitet. Damit können die medizinischen Geräte 6 zentral von der Zentraleinheit 10 mit elektrischer Energie versorgt werden. Die Batterie 5 hat die Aufgabe, bei einem Stromausfall an der Wechselspannungsquelle 3 die Energieversorgung der medizinischen Geräte 6 zu übernehmen. Die Zentraleinheit 10 umfasst ferner eine als Tastatur 17 ausgebildete Bedieneinheit 16. Mittels der Tastatur 17 können die medizinischen

Geräte 6 zentral ein- und ausgeschaltet werden und ferner erfolgt auch die Steuerung oder die Bedienung der medizinischen Geräte 6 zentral von der Tastatur 17 an der Zentraleinheit 10. In die Zentraleinheit 10 ist ferner auch ein Computer 18 eingebaut. Der Computer 18 kann Daten verarbeiten und/oder Speichern, d. h. der Computer 18 verfügt über einen Prozessor, einen Langzeitspeicher (z. B. eine Festplatte) und über einen Arbeitsspeicher. Mit diesem erfindungsgemäßen Aufbau können somit auch mehrere Zentraleinheiten 10 gleichzeitig miteinander in einem medizinischen Gerätesystem 1 betrieben werden.

Die Topologie des Bussystems 12 gemäß der Darstellung in Fig. 1 ist dabei als Stern ausgebildet. An der Zentraleinheit 10 sind sternartig die drei medizinischen Geräte 6 angeschlossen. Abweichend hiervon kann die Topologie des Bussystems 12 auch ein Ring sein (nicht dargestellt).

Die Zentraleinheit 10 überprüft, ob die Summe des elektrischen Leistungsbedarfes der medizinischen Geräte 6 größer ist als die elektrische Leistung, die von der Zentraleinheit 10 zur Verfügung gestellt werden kann. Ist die Summe des elektrischen Leistungsbedarfes der medizinischen Geräte 6 kleiner als die zur Verfügung stellbare elektrische Leistung durch die Zentraleinheit 10, werden alle drei medizinischen Geräte 6 betrieben. Fällt die Wechselspannungsquelle 3 aus, sind die medizinischen Geräte 6 mittels der Batterie 5 in der Zentraleinheit 10 mit elektrischer Energie zu versorgen. Sofern die von der Batterie 5 zur Verfügung stellbare elektrische Energie bzw. die zur Verfügung stellbare elektrische Leistung kleiner ist als die Summe des elektrischen Leistungsbedarfes der drei medizinischen Geräte 6 ist es erforderlich, einzelne medizinische Geräte 6 abzuschalten. Dies wird in der Zentraleinheit 10 anhand der Priorität der medizinischen Geräte 6 vorgenommen. Der Computer 18 der Zentraleinheit 10 schaltet dabei diejenigen medizinischen Geräte 6 mit der niedrigsten Priorität ab, damit die Summe des elektrischen Leistungsbedarfes der im Betrieb befindlichen medizinischen Geräte 6 wieder kleiner ist als die von der Batterie 5 zur Verfügung stellbare elektrische Leistung. Beispielsweise weist das Beatmungsgerät 7 die Priorität eins, der Patientenmonitor 8 die Priorität zwei und der Bildschirm 9 (z.B. zur Anzeige von Labordaten) die Priorität drei auf. Die Zentraleinheit 10 schaltet damit bei einem Ausfall der Wechselspannungsquelle 3 zuerst den Bildschirm 9 ab, so dass der Bildschirm 9 keine elektrische Leistung mehr von der Zentraleinheit 10 benötigt. Es werden somit nur diejenigen medizinischen Geräte 6 mit den höchsten Prioritäten betrieben, so dass die Summe des elektrischen Leistungsbedarfes der in Betrieb befindlichen medizinischen Geräte 6 kleiner als ist als die von der Zentraleinheit 10 zur Verfügung stellbare elektrische Leistung.

Die medizinischen Geräte 6 gemäß der Darstellung in Fig. 1 verfügen über keinen eigenen Gleichrichter 4 und haben auch keine eigene Batterie 5. Abweichend hiervon können medizinische Geräte 6 auch über eine eigene Batterie 5 verfügen zur Versorgung mit elektrischer Energie. Das Vorhandensein einer eigenen Energieversorgung des medizinischen Gerätes 6 wird von dem medizinischen Gerät 6 als technische Daten zur der Zentraleinheit 10 übermittelt.

Beim Anschließen eines weiteren, nicht in Fig. 1 dargestellten medizinischen Gerätes 6 an die Zentraleinheit 10 wird das medizinische Gerät 6 an ein weiteres Interface 14 bzw. Stecker 15 des Bussystems 12 angeschlossen. Dieses zusätzlichen Interface 14 bzw. Stecker 15 sind in der Darstellung in Fig. 1 nicht abgebildet. Beim Anschließen eines medizinischen Gerätes 6 an die Zentraleinheit 10 darf das medizinische Gerät 6 zunächst nur eine sehr geringe elektrische Leistung von der Zentraleinheit 10 aufnehmen. Die elektrische Leistung wird zunächst nur für das Interface 14 an dem medizinischen Gerät 6 verwendet. Das Interface 14 an dem zusätzlichen medizinischen Gerät 6 übermittelt den elektrischen Leistungsbedarf an die Zentraleinheit 10. Nur wenn der elektrische Leistungsbedarf des zusätzlichen medizinischen Gerätes 6 sowie der Leistungsbedarf der bereits vorhandenen medizinischen Geräte 6 die zur Verfügung stellbare elektrische Leistung durch die Zentraleinheit 10 nicht überschreitet, wird das zusätzliche medizinische Gerät 6 in Betrieb genommen bzw. eingeschaltet. Abweichend hiervon können sämtliche medizinischen Geräte 6 auch gemäß der Priorität der medizinischen Geräte 6 (siehe oben) betrieben werden. Das zusätzliche medizinische Gerät 6 kann sich dabei entweder manuell einschalten lassen, z. B. von der Tastatur 17 an der Zentraleinheit 10, oder es schaltet sich selbsttätig ein oder es wartet auf Einschaltdaten von dem Bussystem 12 zum Einschalten nach einer bestimmten Zeit.

Falls die von der Zentraleinheit 10 zur Verfügung stellbare elektrische Energie nicht für das zusätzliche medizinische Gerät 6 ausreicht, wird dieses nicht in Betrieb genommen. Sofern das zusätzliche medizinische Gerät 6 über eine eigene Batterie 5 verfügt, kann das medizinische Gerät trotzdem in Betrieb genommen werden, indem die elektrische Energie für das medizinische Gerät 6 von der Batterie 5 in dem medizinischen Gerät 6 zur Verfügung gestellt wird. Für das medizinische Gerät 6 mit Batterie 5 (nicht dargestellt) sind verschiedene Betriebszustände möglich. Das medizinische Gerät 6 mit Batterie 5 kann beispielsweise betrieben werden mit gleichzeitiger Ladung der Batterie 5, nur mit Ladung der Batterie 5 ohne Betrieb des medizinischen Gerätes 6 betrieben werden oder betrieben werden ohne Ladung der Batterie 5. Diese Betriebszustände des medizinischen Gerätes 6 mit Batterie 5 können zu der Zentraleinheit 10 übermittelt werden und je nach vorhandener zur Verfügung stellbarer elektrischer Leistung von der Zentraleinheit 10 kann ein gerade möglicher und sinnvoller Betriebszustand des medizinischen Gerätes 6 mit Batterie 5 ausgeführt werden. Die Angabe des Betriebszustandes des medizinischen Gerätes 6 erfolgt an einer Anzeigeeinheit (nicht dargestellt) an der Zentraleinheit 10 oder an dem Bildschirm 9.

Das Bussystem 12 dient auch dazu, Daten zwischen den medizinischen Geräten 6 auszutauschen. Dadurch kann der Betrieb der medizinischen Geräte 6 weiter optimiert werden.

Das Bussystem 12 bzw. die Verbindungskabel 13 umfassen beispielsweise wenigstens zwei elektrische Leitungen nur zur Übertragung von Daten und wenigstens zwei Leitungen nur zur Übertragung von elektrischer Energie bzw. zum Leiten von elektrischem Strom. Zur Übertragung von elektrischer Energie gibt es drei Möglichkeiten. Die Spannung in den elektrischen Leitungen zur Übertragung von Energie ist ständig konstant, z. B. auf einem Wert von 24 V. Zweitens in einem Standby-Zustand oder in einem Energiesparmodus sind die Spannungen in den Leitungen zur Übertragung von elektrischer Energie auf einen niedrigeren zweiten Wert als 24 V abgemindert, z. B. auf einen Wert von 5 V. In einer dritten Möglichkeit sind zwei weitere Leitungen vorhanden, welche ständig Strom auf dem niedrigeren Spannungsniveau, z. B. 5 V, liefern und in zwei weiteren Leitungen beträgt die Spannung konstant 24 V.

Die Energieversorgung des medizinischen Gerätesystems kann auch weiter optimiert werden. Beispielsweise wird auch der Ladezustand der Batterie 5 der Zentraleinheit 10 berücksichtigt. Sinkt der Ladezustand der Batterie 5 der Zentraleinheit 10 unter einem bestimmten Wert, z. B. 20 % oder 80 % Ladung, ab werden medizinische Geräte 6 mit einer niedrigen Priorität abgeschaltet. Dies kann auch dahingehend erfolgen, dass je geringer der Ladezustand der Batterie 5 ist, je mehr medizinische Geräte 6 mit einer kleineren Priorität abgeschaltet werden.

Sämtlichen medizinischen Geräte 6 des medizinischen Gerätesystems 1 werden zentral von der Tastatur 17 an der Zentraleinheit 10 ein- und abgeschaltet. Ein gesondertes Ein- und Ausschalten der medizinischen Geräte 6 ist damit nicht mehr einzeln für jedes medizinische Gerät 6 innerhalb des medizinischen Gerätesystems 1 erforderlich. Auch nach dem Abschalten sämtlicher medizinischer Geräte 6 sind das Bussystem 12 bzw. die Interface 14 weiterhin mit elektrischer Energie versorgt, so dass sämtliche medizinischen Geräte 6 des medizinischen Gerätesystems 1 durch ein einzelnes Signal zentral eingeschaltet werden können. Die Energieversorgung des Interfaces 14 bzw. des Bussystems 12 erfolgt auf einem niedrigen Spannungsniveau, z. B. von 5 V. Bei einem Vorhandensein von elektrischen Leitungen (Hauptenergieversorgungsleitungen ) sowohl zur Versorgung auf einem höheren Spannungsniveau, z. B. 24 V, als auch von Leitungen ausschließlich zur Versorgung mit einem niedrigeren Spannungsniveau, z. B. 5 V, können die elektrischen Leitungen zur Versorgung mit dem höheren Spannungsniveau beim Abschalten komplett von der Stromversorgung abgetrennt werden. Dadurch kann in dem Interface 14 die Verlustleistung reduziert werden. Außerdem können bei einem Fehler in einem medizinischen Gerät 6 keine hohen elektrischen Leistungen aus den Hauptenergieversorgungsleitungen mit dem höheren Spannungsniveau entnommen werden. Dadurch kann die Gefahr von Bränden oder anderen schweren Schäden an medizinischen Geräten 6 signifikant reduziert werden.

Die Topologie des Bussystems 12 ermöglicht es auch, festzustellen, ob medizinische Geräte 6 einen Fehler aufweisen. Die Schaltstruktur der Zentraleinheit 10 ist dabei im Allgemeinen dahingehend ausgelegt, dass bei einem Einschalten des medizinischen Gerätesystems 1 zentral an der Tastatur 17 das medizinische Gerät 6 mit dem Fehler nicht eingeschaltet wird. Das Bussystem 12 ist so ausgelegt, dass jeder Teilnehmer des Bussystems, d. h. die medizinischen Geräte 6 und die Zentraleinheit 10, untereinander mit jedem anderen kommunizieren und damit auch Daten austauschen kann. Ein dezidierter Master innerhalb des Bussystems 12 ist nicht erforderlich, jedoch auch nicht hinderlich. Beispielsweise kann die Steuerung so erfolgen, wenn an einem medizinischen Gerät 6 ein Einschaltknopf betätigt wird, dass sämtliche Teilnehmer des Bussystems 12 eingeschaltet werden. Damit lassen sich in besonders einfacher Weise sämtliche Teilnehmer des Bussystems 12 einschalten.

Das Bussystem 12 bzw. die Interface 14 können auch dahingehend ausgelegt sein, dass Daten zu einem medizinischen Gerät 6, z. B. Statusdaten, auch dann sichtbar sind, wenn das medizinische Gerät 6 ausgeschaltet ist. Dies ermöglicht es, dass die Statusinformationen oder technische Daten eines abgeschalteten medizinischen Gerätes an einer Anzeigeeinheit (nicht dargestellt) der Zentraleinheit 10 angezeigt werden oder auch an dem Bildschirm 9. Das Bussystem 12 bzw. die Zentraleinheit 10 kann auch dahingehend ausgelegt werden, dass Software für Teilnehmer, insbesondere medizinische Geräte 6, zur Verfügung gestellt wird. Software für ein medizinisches Gerätesystem 1 braucht damit nur noch zentral auf die Zentraleinheit 10 aufgespielt werden und anschließend wird diese aktualisierte Software als Update den jeweils erforderlichen medizinischen Geräten 6 zur Verfügung gestellt.

Die nicht dargestellte Anzeigeeinheit an der Zentraleinheit 10 oder der Bildschirm 9 sind außerdem dahingehend ausgelegt, dass eine Vereinheitlichung der Benutzeroberfläche möglich ist. Mittels einer Anzeigeeinheit, z. B. der Anzeigeeinheit an der Zentraleinheit 10 und/oder dem Bildschirm 9, können sämtliche Daten der unterschiedlichen medizinischen Geräte 6 angezeigt und visuell dargestellt werden. Die graphische Darstellung unterschiedlicher Daten verschiedener medizinischer Geräte 6 ist damit auf einer einheitlichen Benutzungsoberfläche bzw. Anzeigeeinheit möglich. Dadurch kann das medizinische Gerätesystem 1 besonders einfach und effektiv von dem Benutzer bedient werden.

Insgesamt betrachtet sind mit dem medizinischen Gerätesystem 1 erhebliche Vorteile verbunden. In den medizinischen Geräten 6 können die Kosten für eine Batterie 5 und einen Gleichrichter 4 eingespart werden. Die Batterie 5 und der Gleichrichter 4 sind nur noch zentral in der Zentraleinheit 10 vorhanden. Das Ein- und Ausschalten aller medizinischen Geräte 6 erfolgt zentral von der Bedieneinheit 16 an der Zentraleinheit 10 oder an einem beliebigen medizinischen Gerät des medizinischen Gerätesystems. Damit ist das separate Ein- und Ausschalten sämtlicher medizinischen Geräte 6 nicht mehr erforderlich.

### BEZUGSZEICHENLISTE

- 1: Medizinisches Gerätesystem
- 2: Netzkabel
- 3: Wechselspannungsquelle
- 4: Gleichrichter
- 5: Batterie
- 6: Medizinisches Gerät
- 7: Beatmungsgerät
- 8: Patientenmonitor
- 9: Bildschirm
- 10: Zentraleinheit
- 11: Mittel zur elektrischen Verbindung
- 12: Bussystem
- 13: Verbindungskabel
- 14: Interface
- 15: Stecker
- 16: Bedieneinheit
- 17: Tastatur
- 18: Computer

## Patentansprüche

1. Medizinisches Gerätesystem (1), umfassend
wenigstens ein medizinisches Gerät (6), z. B. ein Beatmungsgerät (7), einen Patientenmonitor (8), ein Anästhesiegerät, ein Inkubator und/oder ein Bildschirm (9),
eine Zentraleinheit (10) zur Stromversorgung des wenigstens einen medizinischen Gerätes (6),
wenigstens ein Mittel (11) zur elektrischen Verbindung der Zentraleinheit (10) mit dem wenigstens einen medizinischen Gerät (6),
**dadurch gekennzeichnet, dass**
die Zentraleinheit (10) eine Batterie (5) und/oder einen Gleichrichter (4) aufweist, so dass das wenigstens eine medizinische Gerät (6) von der Zentraleinheit (10) mit Gleichstrom versorgbar ist.

2. Medizinisches Gerätesystem nach Anspruch 1, **dadurch gekennzeichnet, dass** das medizinische Gerätesystem (1) ein Netzkabel (2) umfasst zum elektrischen Verbinden der Zentraleinheit (10) mit einer Wechselspannungsquelle (3), z. B. mit einer Spannung im Bereich von 110 V oder 220 V, und/oder die Spannung des Gleichstromes zur Versorgung des wenigstens einen medizinischen Gerätes (6) Niederspannung, insbesondere eine Spannung kleiner als 50 V oder 30 V, z. B. im Bereich von 24 V, ist.

3. Medizinisches Gerätesystem nach Anspruch 1 oder 2, **dadurch gekennzeichnet, dass** das wenigstens eine Mittel (11) ein Bussystem (12) und/oder wenigstens ein Verbindungskabel (13) zur elektrischen Verbindung der Zentraleinheit (10) mit dem wenigstens einen medizinischen Gerät (6) und/oder zur Verbindung von wenigstens zwei medizinischen Geräten (6) untereinander umfasst und/oder die Zentraleinheit (10) und/oder wenigstens ein medizinisches Gerät (6) ein Interface (14), z. B. einen Stecker (15), aufweist zur elektrischen Verbindung der Zentraleinheit (10) mit dem wenigstens einen medizinischen Gerät (6) und/oder zur Verbindung von wenigstens zwei medizinischen Geräten (6) untereinander.

4. Medizinisches Gerätesystem nach einem oder mehreren der vorhergehenden Ansprüche, **dadurch gekennzeichnet, dass** Daten von der Zentraleinheit (10) zu dem wenigstens einen medizinischen Gerät (6) übertragbar sind und/oder umgekehrt und/oder Daten zwischen wenigstens zwei medizinischen Geräten (6) übertragbar sind und/oder Daten als elektrische Signale übertragbar sind.

5. Medizinisches Gerätesystem nach einem oder mehreren der vorhergehenden Ansprüche, **dadurch gekennzeichnet, dass** die Zentraleinheit (10) und/oder das wenigstens eine medizinische Gerät (6) wenigstens eine Anzeigeeinheit (9), z. B. einen Bildschirm (9) und/oder wenigstens eine Leuchte, z. B. LED-Leuchte, aufweist und/oder eine Bedieneinheit (16), z. B. wenigstens einen Schalter und/oder eine Tastatur, aufweist.

6. Medizinisches Gerätesystem nach einem oder mehreren der vorhergehenden Ansprüche, **dadurch gekennzeichnet, dass** an der Zentraleinheit (10) wenigstens ein medizinisches Gerät (6), insbesondere sämtliche an der Zentraleinheit (10) angeschlossene medizinischen Geräte (6), ein- und/oder ausschaltbar sind und/oder bedienbar und/oder steuerbar und/oder Daten wenigstens eines medizinischen Gerätes (6) ablesbar sind.

7. Medizinisches Gerätesystem nach einem oder mehreren der vorhergehenden Ansprüche, **dadurch gekennzeichnet, dass** mehrere Zentraleinheiten (10) vorgesehen sind.

8. Medizinisches Gerätesystem nach einem oder mehreren der vorhergehenden Ansprüche, **dadurch gekennzeichnet, dass** wenigstens ein medizinisches Gerät (6) keinen Gleichrichter (4) und/oder keine Batterie (5) aufweist, so dass dem wenigstens einen medizinischen Gerät (6) elektrische Energie ausschließlich von der Zentraleinheit (10) zuführbar ist und/oder ein Verfahren gemäß einem oder mehrerer der Ansprüche 9 bis 15 ausführbar ist.

9. Verfahren zur Stromversorgung eines medizinischen Gerätesystems (1) mit wenigstens einem medizinischen Gerät (6), einer Zentraleinheit (10) zur Stromversorgung des wenigstens einen medizinischen Gerätes (6) und wenigstens einem Mittel (11) zur elektrischen Verbindung der Zentraleinheit (10) mit dem wenigstens einen medizinischen Gerät (6), insbesondere eines medizinischen Gerätesystems (1) gemäß einem oder mehrerer der Ansprüche 1 bis 9, wobei elektrischer Strom von der Zentraleinheit (10) zu wenigstens einem medizinischen Gerät (6) durch das wenigstens eine Mittel (11) geleitet wird, **dadurch gekennzeichnet, dass** Gleichstrom von der Zentraleinheit (10) zu dem wenigstens einen medizinischen Gerät (6) geleitet wird.

10. Verfahren nach Anspruch 10, **dadurch gekennzeichnet, dass** in einem Gleichrichter (4) in der Zentraleinheit (10) Wechselstrom in Gleichstrom umgewandelt wird und/oder in einer Batterie (5) in der Zentraleinheit (10) elektrische Energie gespeichert wird.

11. Verfahren nach Anspruch 11, **dadurch gekennzeichnet, dass** Gleichstrom aus dem Gleichrichter (4) und/oder aus der Batterie (5) zu dem wenigstens einen medizinischen Gerät (6) geleitet wird.

12. Verfahren nach einem oder mehreren der Ansprüche 10 bis 12, **dadurch gekennzeichnet, dass** wenigstens ein medizinisches Gerät (6) Daten, insbesondere hinsichtlich des elektrischen Leistungsbedarfes und/oder der Art oder der Priorität des wenigstens einen medizinischen Gerätes (6), zu der Zentraleinheit (10) übermittelt.

13. Verfahren nach Anspruch 13, **dadurch gekennzeichnet, dass** wenn die Summe des elektrischen Leistungsbedarfes von wenigstens zwei medizinischen Geräten (6) größer ist als die elektrische Leistung der Zentraleinheit (10) zur Versorgung wenigstens eines medizinischen Gerätes (6) von der Zentraleinheit (6) wenigstens ein medizinisches Gerät (6) in einer absteigenden Reihenfolge der Priorität betrieben wird, so dass der elektrische Leistungsbedarf oder die Summe des elektrischen Leistungsbedarfes des wenigstens einen betriebenen medizinischen Gerätes (6) kleiner ist als oder gleich ist wie die elektrische Leistung der Zentraleinheit (10) zur Versorgung des wenigstens einen medizinischen Gerätes (6).

14. Verfahren nach einem oder mehreren der Ansprüche 10 bis 14, **dadurch gekennzeichnet, dass** Daten, z B. Statusinformationen, technische oder medizinische Daten, zu wenigstens einem medizinischen Gerät (6), vorzugsweise sämtlichen medizinischen Geräten (6), an der Zentraleinheit (10) oder einem medizinischen Gerät (6) angezeigt werden, z. B. an einem Bildschirm (9) oder wenigstens einer Leuchte, z. B. LED-Leuchte, insbesondere Daten zu wenigstens zwei medizinischen Geräten (6) an nur einer Anzeigeeinheit (9), z. B. Bildschirm (9), angezeigt werden.

15. Verfahren nach einem oder mehreren der Ansprüche 10 bis 15, **dadurch gekennzeichnet, dass** wenigstens ein medizinisches Gerät (6), insbesondere sämtliche an der Zentraleinheit (10) angeschlossene medizinischen Geräte (6), an der Zentraleinheit (10) ein- und ausschaltet werden und/oder bedient werden und/oder gesteuert werden und/oder Daten wenigstens eines medizinischen Gerätes (6) abgelesen werden.
